# EUROPÄISCHE PATENTANMELDUNG

(11) **EP 0 916 665 A1**
(43) Veröffentlichungstag der Anmeldung: **19.05.1999**
(21) Anmeldenummer: 98120759.0
(22) Anmeldetag: 02.11.1998
(51) Int. Cl.: C07D 311/72, C07D 311/70

(54) **Verfahren zur Herstellung von Chromanderivaten**

(30) Priorität: 11.11.1997 CH 2601/97
(71) Anmelder: LONZA A.G., CH-4002 Basel (CH)
(72) Erfinder: Jackson, Barry, 3902 Brig-Glis (CH)

(57) **Zusammenfassung**

Chromanderivate der allgemeinen Formel 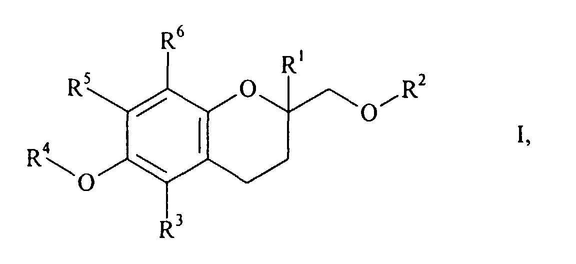 worin R¹ Wasserstoff oder C₁₋₄-Alkyl; R² C₁₋₆-Alkyl, C₃₋₆-Cycloalkyl, gegebenenfalls substituiertes Aryl oder gegebenenfalls substituiertes Aryl-C₁₋₆-alkyl; R³, R⁵ und R⁶ unabhängig voneinander Wasserstoff, Halogen, C₁₋₆-Alkyl oder gegebenenfalls substituiertes Aryl; und R⁴ C₁₋₆-Alkyl, C₃₋₆-Cycloalkyl, (C₁₋₆-Alkoxy)methyl, gegebenenfalls substituiertes Aryl, gegebenenfalls substituiertes Aryl-C₁₋₆-alkyl, C₁₋₆-Alkanoyl oder gegebenenfalls substituiertes Aroyl bedeuten, werden aus Hydrochinonderivaten und Alkenen in Gegenwart von Lewis-Säuren erhalten. Die Chromanderivate (I) sind Zwischenprodukte in der Synthese von pharmazeutischen Wirkstoffen, beispielsweise von Lipidsenkern.

## Beschreibung

Die vorliegende Erfindung betrifft ein Verfahren zur Herstellung von Chromanderivaten der allgemeinen Formel

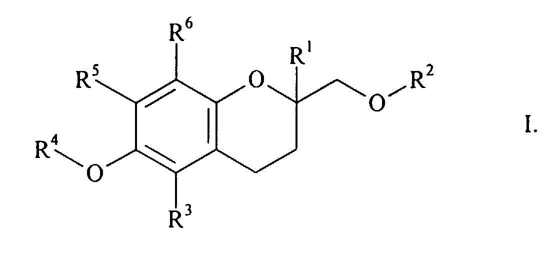

Hierin bedeuten:
R¹ Wasserstoff oder C₁₋₄-Alkyl;
R² C₁₋₆-Alkyl, C₃₋₆-Cycloalkyl, gegebenenfalls substituiertes Aryl oder gegebenenfalls substituiertes Aryl-C₁₋₆-alkyl;
R³, R⁵ und R⁶ unabhängig voneinander Wasserstoff, Halogen, C₁₋₄-Alkyl oder gegebenenfalls substituiertes Aryl;
R⁴ C₁₋₆-Alkyl, C₃₋₆-Cycloalkyl, (C₁₋₆-Alkoxy)methyl, gegebenenfalls substituiertes Aryl, gegebenenfalls substituiertes Aryl-C₁₋₆-alkyl, C₁₋₆-Alkanoyl oder gegebenenfalls substituiertes Aroyl.

Verbindungen dieser Art sind Zwischenprodukte für die Synthese von pharmazeutischen Wirkstoffen, z. B. von Lipidsenkern (T. Yoshioka ei al., *J. Med. Chem.* **1989**, *32*, 421-428).

Bekannte Synthesen der Chromanderivate (I) gehen von entsprechend substituierten Hydrochinonen aus, beispielsweise von 3-(2,5-Dihydroxyphenyl)propan-1-olen (siehe z. B.: *Comprehensive Heterocyclic Chemistry* (Ed.: A. J. Boulton, A. McKillop), Pergamon Press, Oxford, Vol. 3, Part 2B, S. 778ff).

Aus den daraus durch Cyclodehydratisierung erhältlichen Chroman-2-carbonsäureestern sind durch Reduktion der Esterfunktion und Veretherung der so erhaltenen Hydroxymethylgruppe die Titelverbindungen erhältlich. Das Verfahren ist jedoch ziemlich umständlich.

Aufgabe der vorliegenden Erfindung war daher, ein alternatives Herstellungsverfahren bereitzustellen, das in wenigen Stufen die gewünschten Verbindungen (I) liefert. Erfindungsgemäss wird diese Aufgabe durch das Verfahren nach Patentanspruch 1 gelöst.

Es wurde gefunden, dass aus den Hydrochinonderivaten der allgemeinen Formel 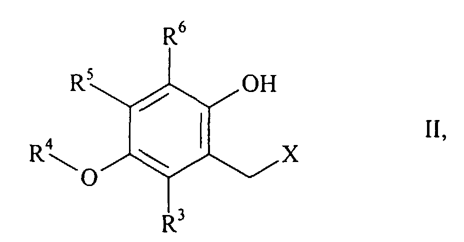 worin R³ bis R⁶ die oben genannten Bedeutungen haben und X eine Abgangsgruppe ist, durch Umsetzung mit Alkenen der allgemeinen Formel 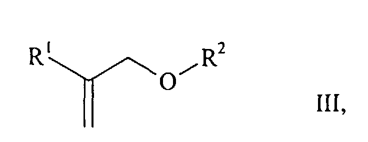 worin R¹ und R² die oben genannten Bedeutungen haben, in Gegenwart einer Lewis-Säure in nur einer Stufe die Titelverbindungen (I) hergestellt werden können.

Das erfindungsgemässe Verfahren verläuft wahrscheinlich über ein *o-*Chinonmethid (P. Wan et al., *Can. J. Chem.* **1996**, *74*, 465-475) als Zwischenstufe.

Die Hydrochinonderivate (II) sind bekannte Verbindungen oder können analog zu bekannten Verbindungen hergestellt werden.

Als Abgangsgruppen eignen sich beispielsweise Halogen, insbesondere Chlor oder Brom, Hydroxy, oder Esterfunktionen wie Alkanoat oder Sulfonat, also beispielsweise Acetat oder Tosylat. Mögliche Synthesewege sind beispielsweise die Halomethylierung der entsprechenden Hydrochinone, die Seitenkettenhalogenierung der entsprechenden Methylverbindungen oder der Austausch der Hydroxygruppe in den entsprechenden Hydroxymethylverbindungen gegen Halogen, Alkanoat oder Sulfonat.
Die Hydroxymethylverbindungen können beispielsweise durch Hydroxymethylierung der entsprechenden Hydrochinone oder durch Reduktion der entsprechenden Aldehyde oder Carbonsäurederivate hergestellt werden.
Ebenso sind die Alkene (III) bekannte Verbindungen oder können analog zu bekannten Verbindungen hergestellt werden. Da diese eine Etherfunktion enthalten, kommen zu ihrer Herstellung die üblichen Methoden zur Herstellung von unsymmetrischen Ethern in Frage, beispielsweise die Williamson-Synthese und verwandte Reaktionen.

Unter C_{1-*n*}-Alkyl sind hier und im folgenden jeweils alle primären, sekundären und tertiären linearen oder verzweigten Alkylgruppen mit 1 bis *n* Kohlenstoffatomen zu verstehen, unter C₁₋₆-Alkyl also beispielsweise Methyl, Ethyl, Propyl, Butyl, Pentyl, Hexyl, Isopropyl, Isobutyl, Isopentyl, Isohexyl, *sec*-Butyl, *tert*-Butyl, Neopentyl usw. Entsprechend sind unter C₁₋₆-Alkoxy und C₁₋₆-Alkanoyl die aus C₁₋₆-Alkyl und Sauerstoff bzw. die aus Wasserstoff oder C₁₋₅-Alkyl und Carbonyl zusammengesetzten Gruppen zu verstehen.
Unter C₃₋₆-Cycloalkyl sind Cyclopropyl, Cyclobutyl und insbesondere Cyclopentyl und Cyclohexyl zu verstehen.
Unter Aryl sind mono- und polycyclische carbo- und heterocyclische aromatische Reste zu verstehen, also beispielsweise Phenyl, Naphthyl, Pyridyl, Furyl, Thiophenyl, Pyrrolyl oder Indolyl. Entsprechend sind unter Aroyl die aus Aryl und Carbonyl zusammengesetzten Gruppen zu verstehen.
Die Aryl- oder Aroylgruppen können gegebenenfalls einen oder mehrere gleiche oder verschiedene Substituenten tragen, insbesondere Gruppen wie Halogen, C₁₋₄-Alkyl, C₁₋₄-Perfluoralkyl, C₁₋₄-Alkoxy, Nitro oder C₁₋₄-Alkanol. Grundsätzlich sind alle Substituenten möglich, die unter den Reaktionsbedingungen stabil sind.

Vorzugsweise werden nach dem erfindungsgemässen Verfahren diejenigen Chromanderivate (I) hergestellt, in denen R¹ eine Methylgruppe ist. Die in diesem Fall als Edukt erforderlichen Methallylether (III, R¹ = Me) können beispielsweise aus dem kommerziell erhältlichen Methallylchlorid hergestellt werden.
Ebenfalls bevorzugt werden diejenigen Chromanderivate hergestellt, in welchen R² eine gegebenenfalls substituierte Arylgruppe ist.
Die in diesem Fall als Edukt dienenden Arylether (III, R² = Aryl) können beispielsweise aus den entsprechenden Alkaliphenolaten und einem entsprechenden Alkenylhalogenid hergestellt werden.

Besonders bevorzugt ist die Herstellung von Chromanderivaten (I), in denen R² *p-*Nitrophenyl oder *p-*Formylphenyl ist.

Ebenfalls bevorzugt ist die Herstellung von Chromanderivaten (I), in denen R³, R⁵ und R⁶ Methylgruppen sind. Das hierfür als Ausgangsmaterial erforderliche Hydrochinonderivat (II, R³ = R⁵ = R⁶) kann aus dem kommerziell erhältlichen Trimethylhydrochinon synthetisiert werden.

Desgleichen ist die Herstellung von denjenigen Chromanderivaten (I), in denen R⁴ eine Acetylgruppe ist, bevorzugt.

Als Hydrochinonderivat (II) wird vorzugsweise eine solches eingesetzt, in dem X Chlor ist, also eine Chlormethylverbindung.

Als Lewis-Säure kann grundsätzlich jede der in der organischen Synthese als Katalysator üblichen Lewis-Säuren eingesetzt werden, bevorzugt sind diejenigen aus der Gruppe, die aus Bortrifluorid, Aluminiumchlorid, Titan( 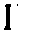 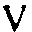 )chlorid, Scandium-trifluormethansulfonat, Eisen( 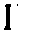 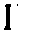 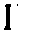 )chlorid, Zirconium( 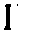 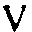 )chlorid und Zinn( 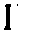 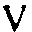 )chlorid gebildet wird.

Als Lösungsmittel eignen sich insbesondere aprotische Lösungsmittel geringer bis mittlerer Polarität wie beispielsweise halogenierte Kohlenwasserstoffe, Ether oder Ester.

Bevorzugt sind Dichlormethan, Chloroform, Tetrahydrofuran und Ethylacetat sowie deren Gemische.

Die Reaktion wird vorteilhaft bei einer Temperatur von 0 bis 100 °C durchgeführt, vorzugsweise bei 20 bis 80 °C.
Die nachfolgenden Beispiele verdeutlichen die Durchführung des erfindungsgemässen Verfahrens, ohne dass darin eine Einschränkung zu sehen ist.

### Beispiele

### Beispiel 1

### 6-Acetoxy-2,5,7,8-tetramethyl-2-[(4-nitrophenoxy)methyl]chroman (I, R¹ = R³ = R⁵ = R⁶ = Me, R² = 4-C₆H₄NO₂, R⁴ = Ac)

In 18,2 g Dichlormethan wurden unter Stickstoff 2,43 g (10 mmol) 4-Acetoxy-2-(chlormethyl)-3,5,6-trimethylphenol (hergestellt gemäss L. I. Smith, R. B. Carlin, *J. Am. Chem. Soc.* **1942**, *64*, 524-527) und 2,13 g (11 mmol) Methallyl-(4-nitrophenyl)-ether (hergestellt gemäss DE-A-19 25 112) suspendiert. Dann wurden 0,25 g (0,5 mmol) Scandium-trifluormethansulfonat (Fluka) zugegeben und das Gemisch 1,5 h zum Rückfluss erhitzt. Anschliessend wurde das Reaktionsgemisch mit 16 g Eis versetzt und die wässrige Phase abgetrennt. Die organische Phase wurde mit 20 ml Wasser und dann mit 4 × 20 ml gesättigter Natriumhydrogencarbonatlösung gewaschen, mit Natriumsulfat getrocknet und am Rotationsverdampfer eingeengt. Der getrocknete Rückstand (3,53 g) hatte gemäss HPLC-Analyse einen Gehalt von 71,9%.
Ausbeute: 63,6% (bezogen auf die Chlormethylverbindung).
Das Rohprodukt wurde in 20 g siedendem Ethanol gelöst, die Lösung langsam auf Raumtemperatur abgekühlt und dann noch 3 h bei 5 °C gehalten. Dann wurde das ausgefallene Produkt über eine Glasfilternutsche abfiltriert, mit ca. 2 ml kaltem Ethanol gewaschen und getrocknet. Der Gehalt des so umkristallisierten Produkts war gemäss ¹H NMR ca. 98%. Zur weiteren Reinigung und Charakterisierung wurde eine Probe mit Hexan/Ethylacetat (6:1) an Aluminiumoxid chromatographiert.
Schmp.: 128 °C (aus Cyclohexan).
¹H NMR (CDCl₃): δ 8,19/6,98 (AA'XX', 4H); 4,11/4,01 (AB, *J* = 3 Hz, 2H); 2,65 (m, 2H); 2,34 (s, 3H); 2,14 (m, 1H), 2,05 (s, 3H); 2,02 (s, 3H); 1,98 (s, 3H); 1,90 (m, 1H); 1,43 (s, 3H).

### Beispiel 2

### 4-[(6-Acetoxy-2,5,7,8-tetramethylehroman-2-yl)methoxy]benzaldehyd (I, R¹ = R³ = R⁵ = R⁶ = Me, R² = 4-C₆H₄CHO, R⁴ = Ac)

Unter Stickstoff wurden 38,32 g (150 mmol, 95%ig) 4-Acetoxy-2-(chlormethyl)-3,5,6-trimethylphenol und 25,11 g (142,5 mmol) 4-(2-Methylprop-2-enyloxy)benzaldehyd in 217,4 g Dichlormethan suspendiert. Dann wurden 1,90 g (3,8 mmol) Scandium-trifluormethansulfonat zugegeben, worauf sich in leicht exothermer Reaktion eine dunkelrote Suspension bildete. Diese wurde insgesamt ca. 23 h am Rückfluss gekocht, wobei nach 5 h 5 g Aktivkohle zugesetzt wurden. Anschliessend wurde das Reaktionsgemisch filtriert und nacheinander mit je 100 ml Wasser, gesättigter Natriumhydrogencarbonatlösung und 10%iger Natriumchloridlösung gewaschen. Die organische Phase wurde über Natriumsulfat getrocknet und am Rotationsverdampfer eingeengt. Es blieben 58,08 g eines öligen Rohprodukts mit einem Gehalt von ca. 60% zurück, was einer Ausbeute von 61% (bezogen auf die Chlormethylverbindung) entspricht. Zur Reinigung wurde das Rohprodukt mit Hexan/Ethylacetat (ν:ν = 6:1) an Aluminiumoxid chromatographiert und nach dem Einengen des Eluats in Ethanol aufgeschlämmt, filtriert und getrocknet.
Schmp.: 89 °C
¹H NMR(CDCl₃): δ 9,87 (s, 1H); 7,82 (m, 2H); 7,03 (m, 2H); 4,10 (d, *J* = 10 Hz, 1H); 3,98 (d, *J* = 10 Hz, 1H); 2,64 (m, 2H); 2,34 (s, 3H); 2,13 (m, 1H); 2,07 (s, 3H); 2,02 (s, 3H); 1,99 (s, 3H); 1,89 (m, 1H); 1,44 (s, 3H).
IR ν̃ 1785; 1692 cm⁻¹.
MS [*m/z*] 382 (M⁺, 24%); 340 (100); 219 (15); 205 (61); 203 (15); 191 (13); 165 (50); 121 (12); 91 (16); 43 (49).

## Patentansprüche

1. Verfahren zur Herstellung von Chromanderivaten der allgemeinen Formel 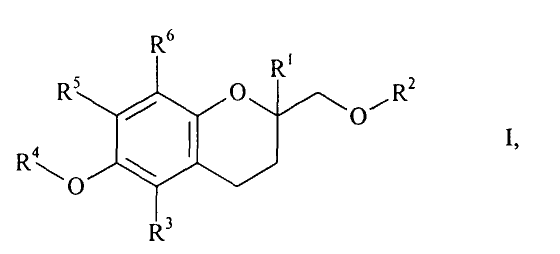
worin R¹ Wasserstoff oder C₁₋₄-Alkyl,
R² C₁₋₆-Alkyl, C₃₋₆-Cycloalkyl, gegebenenfalls substituiertes Aryl oder gegebenenfalls substituiertes Aryl-C₁₋₆-alkyl,
R³, R⁵ und R⁶ unabhängig voneinander Wasserstoff, Halogen, C₁₋₄-Alkyl oder gegebenenfalls substituiertes Aryl und
R⁴ C₁₋₆-Alkyl, C₃₋₆-Cycloalkyl, (C₁₋₆-Alkoxy)methyl, gegebenenfalls substituiertes Aryl, gegebenenfalls substituiertes Aryl-C₁₋₆-alkyl, C₁₋₆-Alkanoyl oder gegebenenfalls substituiertes Aroyl bedeuten, dadurch gekennzeichnet, dass ein Hydrochinonderivat der allgemeinen Formel 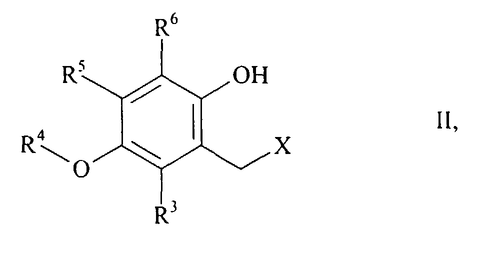 worin R³ bis R⁶ die oben genannten Bedeutungen haben und X eine Abgangsgruppe ist, in Gegenwart einer Lewis-Säure mit einem Alken der allgemeinen Formel 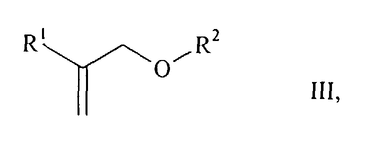 worin R¹ und R² die oben genannten Bedeutungen haben, umgesetzt wird.

2. Verfahren nach Anspruch 1, dadurch gekennzeichnet, dass R¹ Methyl ist.

3. Verfahren nach Anspruch 1 oder 2, dadurch gekennzeichnet, dass R² gegebenenfalls substituiertes Aryl ist.

4. Verfahren nach Anspruch 3, dadurch gekennzeichnet, dass R² *p-*Nitrophenyl oder *p-*Formylphenyl ist.

5. Verfahren nach einem der Ansprüche 1 bis 4, dadurch gekennzeichnet, dass R³, R⁵ und R⁶ Methylgruppen sind.

6. Verfahren nach einem der Ansprüche 1 bis 5, dadurch gekennzeichnet, dass R⁴ Acetyl ist.

7. Verfahren nach einem der Ansprüche 1 bis 6, dadurch gekennzeichnet, dass X Chlor ist.

8. Verfahren nach einem der Ansprüche 1 bis 7, dadurch gekennzeichnet, dass als Lewis-Säure eine Verbindung aus der Gruppe bestehend aus Bortrifluorid, Aluminiumchlorid, Titan( 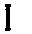 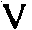 )chlorid, Scandium-trifluormethansulfonat, Eisen( 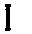 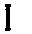 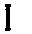 )chlorid, Zirconium( 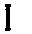 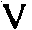 )chlorid und Zinn( 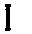 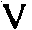 )chlorid eingesetzt wird.

9. Verfahren nach einem der Ansprüche 1 bis 8, dadurch gekennzeichnet, dass die Umsetzung in einem Lösungsmittel aus der Gruppe bestehend aus Dichlormethan, Chloroform, Tetrahydrofuran und Ethylacetat durchgeführt wird.
